# EUROPEAN PATENT APPLICATION

(11) **EP 2 752 795 A1**
(43) Date of publication of application: **09.07.2014**
(21) Application number: 13150461.5
(22) Date of filing: 08.01.2013
(51) Int. Cl.: G06Q 10/06

(54) **Diagnostic workflow management with pluggable report sections**

(71) Applicant: Agfa Healthcare, 2640 Mortsel (BE)
(72) Inventor: Duwyn, Ronny, 2640 Mortsel (BE); Vanheuverswyn, Jeroen, 2640 Mortsel (BE); Kieckens, Wannes, 2640 Mortsel (BE)

(57) **Abstract**

A method of operating a data processing system for diagnostic workflow management with generation of sectional reports (200) comprises defining an extension point for report sections enabling users to create one or more independent pluggable components each of which implements a user-configurable report section (104, 204, 306, 406, 502).

## Description

### Field of the Invention

The present invention generally relates to computer implemented methods for reporting in healthcare applications, more particularly in diagnostic workflows. The invention in particular concerns the ability for the customer, e.g. the hospital or medical imaging centre, to customize and/or extend sectional reports.

### Background of the Invention

Development of applications in healthcare like diagnostic workflow management applications faces several challenges like a fast evolving market and fast evolving technology. Such applications must therefore be made future-proof. Where a reporting module is common to most diagnostic workflow management applications, there are a lot of differences that make the reporting module often unique per customer/installation. The main differences reside in how the reporting is done, e.g. text entry or speech recognition, and in the capabilities of customizing and extending the reporting. In current applications, the options the customer gets for customizing and/or extending the repoprting are limited to what the application can handle, i.e. limited to what has been developed upfront in the application.

Prior art solutions for medical reporting generally can be classified in two categories: non-sectional medical reporting and sectional medical reporting.

In non-sectional medical reporting, the reporting is done in on piece. This means that all report content is entered by the report author as one big block of data. The order and format of that data is defined by how the author enters it. This can be done by opening an application, e.g. a text editor like Microsoft Word, manually entering the text, tables and images, and entering headers and footers.

In sectional reporting, the report author enters content in different parts, the so called sections. Business rules and report structures determine how the different sections are combined. This method of reporting has the advantage of being very flexible, less time consuming and less error-prone. Sectional medical reports have a fixed structure. The user defines the sections that form part of the report, as well as their location and processing. An example of such method for sectional reporting is described in United States Patent Application US 2010/0229084 entitled "Rapid Software Report Development Tool with Auto Database Handling and Menu Tool-Bar Plug-In for Application Report Access". Therein, a computer implemented method is described for creating a sectional report that consists in making a selection of fields or sections to be included in the report, determining the position of these fields in a layout, associating the sections or fields with data sources in a database, and generating a displayed report and a printed report in accordance with the layout.

The known method for sectional reporting is disadvantageous in that the sections and their position in the layout need to be defined upfront. The format, location and processing of each of the sections in other words is predefined. The customer consequently cannot introduce extensions or customized sections in a medical report without returning to the developer of the diagnostic workflow application where the reporting module forms part of.

Another example method for medical report creation is known from United States Patent Application US 2007/0050701 entitled "Method, System and Computer Program Product for Medical Form Creation". This US patent application describes a method wherein a sectional medical form is defined by a plurality of components or so-called operands. Although a number of operands are modifiable upon form completion, the medical forms in US 2007/0050701 cannot be customized or extended with user-configured sections. As a consequence, the method known from US 2007/0050701 is not future-proof.

It is an objective of the present invention to disclose a computer implemented method for diagnostic workflow management with generation of sectional reports that overcomes the above mentioned drawbacks of existing methods. More particularly, it is an objective of the present invention to provide a computer implemented method for diagnostic workflow management with generation of sectional reports that enables the customer to autonomously introduce extensions and customized sections. The invention in other words aims at providing a method and tool for medical reporting that is open to customers that for instance have their own IT department able to define inexistent sections, like for instance a particular image, video, table, etc.

### Summary of the Invention

According to the present invention, the above defined objectives are realized by the method of operating a data processing system for diagnostic workflow management with generation of sectional reports as defined by claim 1, the method comprising defining an extension point for report sections enabling users to create one or more independent pluggable components each of which implements a user-configurable report section.

Thus, the present invention provides an extension point enabling the customer, e.g. the hospital or medical imaging centre, to create plug-in components that can be integrated in a report. The components can be any existing or future developed components, such as for instance questionnaires, images, moving images, etc. Thanks to the extension point, the medical reporting becomes customer-customizable and future-proof. The customer or external companies different from the developer of the diagnostic workflow management application can create and commercialize the plug-ins needed to create certain sections in the reports.

As further defined by claim 2, the extension point in the method according to the present invention represents a public interface comprising a title for configuring a title of a report section, a display visualisation for configuring visualization of the report section on a display, and a report visualisation for configuring visualization of the report section in a report.

Thus, the extension point that interfaces between the diagnostic workflow management application and a report plugin, at least defines the title of the section, the way the section should be visualized in a displayed version of the report on screen, and the way the section should be displayed in a printed version of the report.

As further specified by claim 3, the report section may represent one or more of:
- a questionnaire;
- a still image;
- moving images.

It is noticed that the above list of possible customer-created report sections that can be plugged-in through the present invention is not exhaustive.

In addition to a computer implemented method as defined by claim 1, the present invention relates to a corresponding data processing system as defined by claim 4, comprising means for carrying out such a method.

Further, the present invention also relates to a corresponding computer program as defined by claim 5, comprising software code adapted to perform such a method.

The present invention also relates to a corresponding computer readable storage medium as defined by claim 6, comprising the computer program.

### Brief Description of the Drawings

Fig. 1 shows a screenshot or displayed visualization in a first embodiment of the method according to the present invention wherein a user-configured questionnaire is plugged-in as a separate section;

Fig. 2 is a printed visualization of a medical report in the first embodiment of the method according to the present invention wherein the filled-out user-configured questionnaire is integrated in the medical report;

Fig. 3 shows a screenshot or displayed visualization in a second embodiment of the method according to the present invention wherein a user-configured image of the examined body part is plugged-in as a separate section;

Fig. 4 illustrates configuration of the plug-in section containing an image of the examined body part in the second embodiment; and

Fig. 5 illustrates integration of the plug-in section containing an image of the examined body part in the medical report structure for the second embodiment.

### Detailed Description of Embodiment(s)

The invention generally enables customers of a diagnostic workflow management application to create independent pluggable components that can easily integrate in the medical reports generated by such application. The components could for instance be questionnaires as will be illustrated in a first embodiment described below, images as will be illustrated in a second embodiment described below, movies, tables, or any other visual or non-visual component that a customer of such application would like to integrate in a medical report now or in the future.

It is important that the hosting diagnostic workflow management application is built upon an architectural pattern that enables building an application from constituent parts, like for instance the Eclipse plug-in framework. The basic mechanism of extensibility in Eclipse is that new plug-ins can add new processing elements to existing plug-ins. Eclipse provides a set of core plug-ins to bootstrap this process.

Embodiments of the present invention make use of the above mechanism by defining an extension point for report sections enabling customers to create pluggable report components via implementations of the extension point: <extension-point id="section" name="section" schema="schema/section.exsd"/>

Each customer-configured medical report component requires an implementation of the above extension point. An example is for instance:

```
 <extension point="com.agfa.ris.client.textfields.section">
      <section class=
      "com.agfa.ris.client.textfields.extendedSection.ProtoTypeSection"
      id="PROTOTYPE"
      name="PROTOTYPE">
 </section>
```

Thus, it is required to provide the qualified class name, a unique identifier and a unique name for an implementation of the extension point.

The extension point schema defines that the extension points 'section' class needs to implement the ISection interface:
public interface ISection {
   public JComponent getPanel();
      This method returns a component that displays the user-configurable report section.
   public ReportSectionContent getContent();
      This method returns the structured data that has been entered in the user-configurable report section or is available by default through setup.
   public boolean hasContent();
      This method indicates whether the user-configurable report section contains some content to know whether the report has changed.
   public void loadContent(ReportSectionContent content);
      This method loads the given content into the user-configurable report section.
   public void setComponentName(String name);
      This method gives a name to the component.
   public boolean hasFocus();
      This method checks whether the component has the input (mouse or keyboard) focus.
   public boolean requestFocus();
      This method requests mouse or keyboard focus.
   public boolean isFocusable();
      This method checks whether the component can take focus, i.e. it defines the tab sequence such that for instance a particular image is just shown in the user-configurable report section and need not be edited.
   public void addSectionListener(ISectionListener sectionListener);
      This method registers a listener that for instance needs to be notified of changes in the user-configurable report section.
   public void removeSectionListener(ISectionListener sectionListener);
      This method unregisters a listener object.
   public void addMouseListener(MouseListener mouseListener);
      This method registers a mouse listener that needs to be notified on mouse clicks or movements with the component.
   public void removeMouseListener(MouseListener mouseListener);
      This method unregisters a mouse listener object.
   public void reloadAutoContent(Context context);
      This method loads a pre-set or pre-configured data, and may be optional.
   public void clear();
      This method clears the user-configurable report section.
   public void prepareForRecycle();
      This method releases memory and objects such that the component doesn't need to be recreated when a new report is loaded.

This 'prototype' section PrototypeSection implements this interface so that it will be able to interact with the hosting application.

When the application finds the extensions, the reporting configuration will dynamically show the configuration capabilities of the installed plug-in. By including such a report section into a report structure, it will become available to the user doing the reporting.

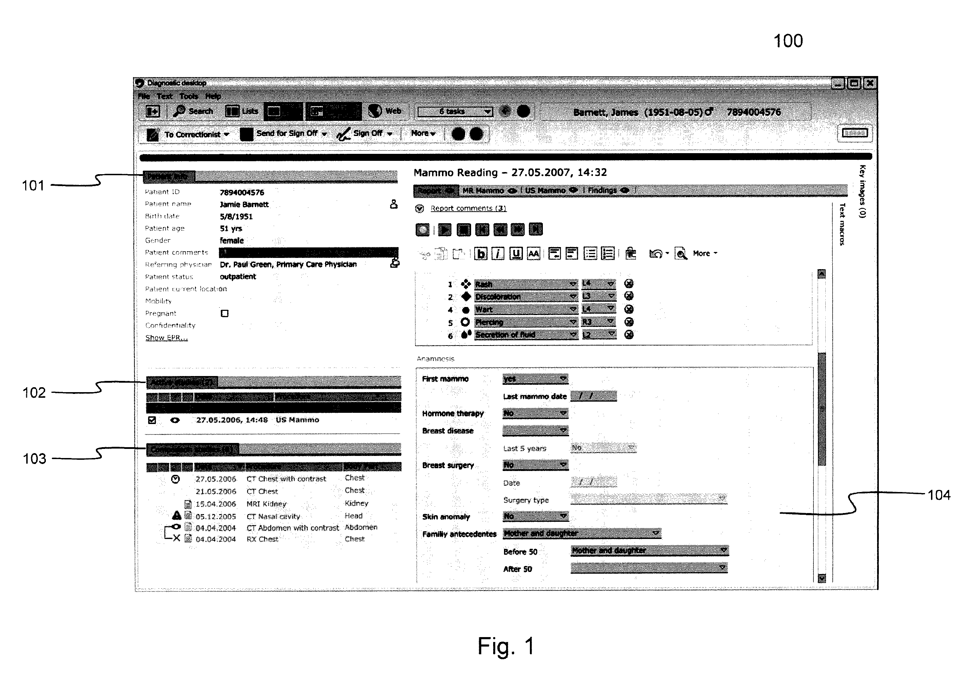

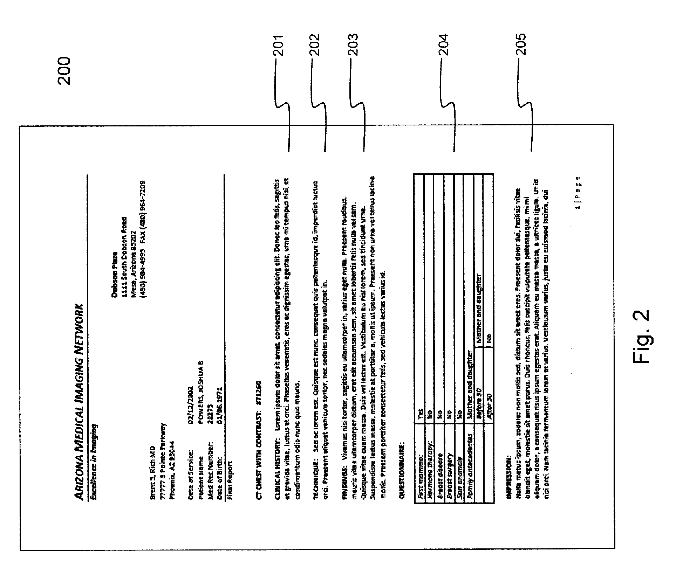

In a first embodiment of the invention, illustrated by Fig. 1 and Fig. 2, a user-configured questionnaire is integrated in a medical report for mammography.

Fig. 1 shows a screenshot 100 displayed by the diagnostic workflow management application to the physician. The screenshot shows on the left side general information like patient information 101, information 102 on active studies or examinations the patient is undergoing, and information 103 on earlier or comparison studies where the patient has been subject to. On the right side, the screenshot shows the sections that will be integrated in the medical report on the mammography. The section 104 shows that a user-configured questionnaire can be integrated. This user-configured questionnaire has been inserted via a plug-in that implements the extension point thereto foreseen in the hosting diagnostic workflow management application.

Fig. 2 further illustrates how the user-configurable questionnaire is integrated in the medical report 200 on the mammography. The medical report contains a number of pre-programmed sections like a section 201 containing information on the clinical history of the patient, a section 202 on the technique applied for examining the patient, a section 203 wherein the physician describes his findings and a section 205 wherein the physician describes his impression. In addition to the pre-programmed sections, the medical report 200 also contains a section 204 containing the responses to the user-configured questionnaire. The results of the questionnaire are integrated in the form of a table in the medical report 200 on the mammography. This is made possible since the plug-in implementing the user-configurable questionnaire configures not only the visualization on the display but also the visualization in the medical report.

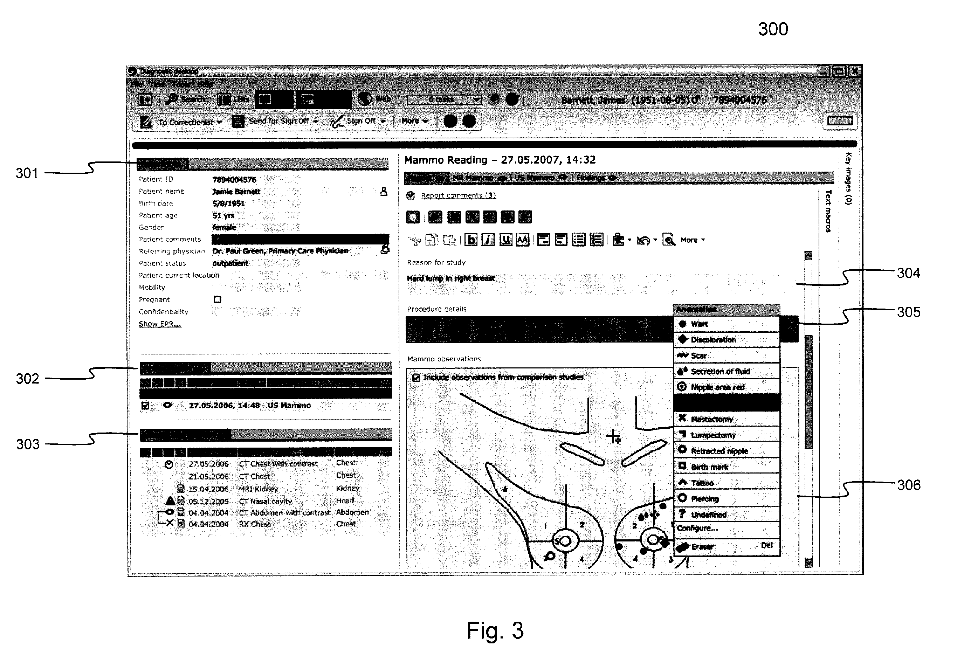

In a second embodiment of the invention, illustrated by Fig. 3, Fig. 4 and Fig. 5, an image of the examined body part is integrated in the medical report on mammography. The image can be used by the technician to mark observable anomalies like for instance the presence of a wart, a birth mark, a tattoo, a piercing, etc. in order to ease examination by the physician. Again the image that can be marked by the technician is not a standard component of the diagnostic workflow management application. Instead, it is integrated in the displayed screenshots and the printable medical report through a customer-generated plug-in that implements the extension point for medical reports that is foreseen in the hosting application.

Fig. 3 shows a screenshot 300 displayed by the diagnostic workflow management application to the technician during the mammography. The screenshot shows on the left side general information like patient information 301, information 302 on active studies or examinations the patient is undergoing, and information 303 on earlier or comparison studies where the patient has been subject to. On the right side, the screenshot shows the sections that will be integrated in the medical report on the mammography, like for instance a section 304 containing information on the reason for the study, a section 305 containing information on the procedure and a section 306 containing an image of the examined body part and a table enabling the technician to mark on the image observable anomalies. This customer-configured image has been inserted in section 306 via a plug-in that implements the extension point thereto foreseen in the hosting diagnostic workflow management application.

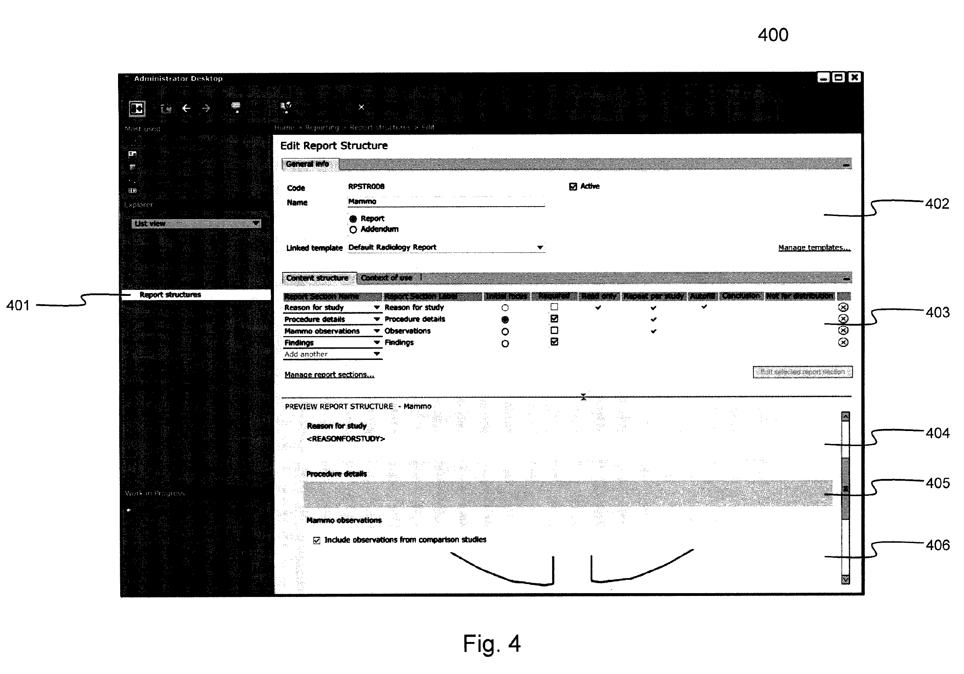

Fig. 4 further illustrates configuration of medical report and more particularly the plug-in section containing the image of the examined body part in the administrator desktop 400 of the hosting application. In the administrator desktop 400, the menu on the left side contains an item 401 that enables to edit the medical report structure. The user there configures the medical report by selecting the default radiology report structure in 402. In table 403, the user can indicate which sections need to be integrated in the report. The table not only lists the default, pre-programmed sections like "Reason for study", "Procedure details" and "Findings" but also lists the components that are available through customer configured plug-ins like "Mammo observations", i.e. the image of the body part that enables the technician to mark observations. As a result of the configuration, the medical report structure shall be as shown in the preview, i.e. it shall contain a section 404 for information detailing the reason of the study, a section 405 for information on the procedure details, and a section 406 for the image of the examined body part.

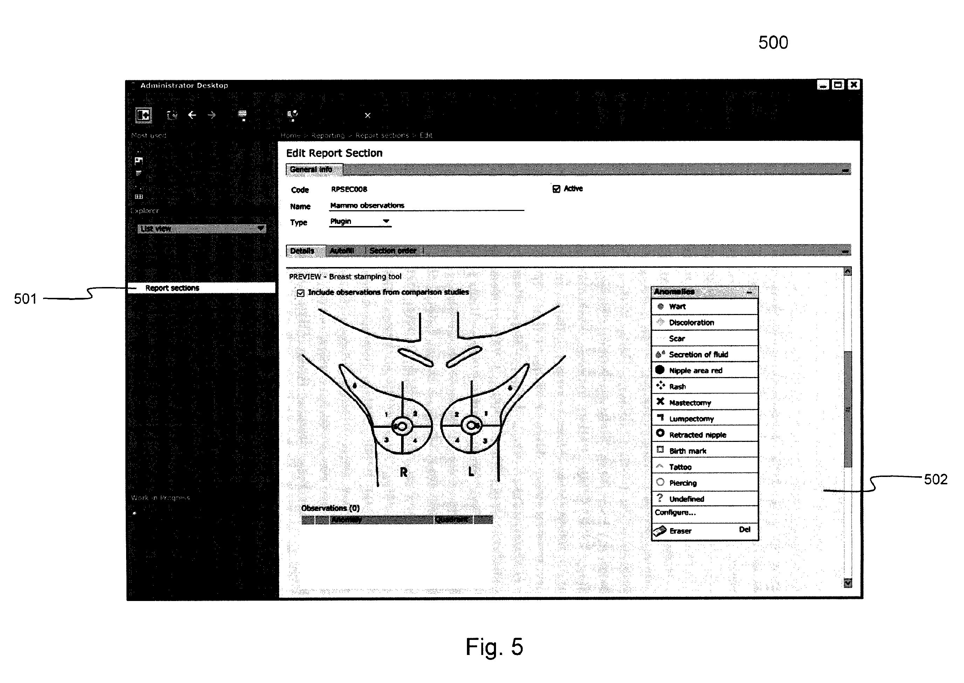

Fig. 5 at last illustrates how the section containing the image of the examined body part 502 will be integrated in the structure of the medical report. Thereto, the "Report sections" 501 must be selected in the administrator desktop 500.

It is noticed that the mechanism according to the invention is built upon an existing plug-in framework. Thanks to the extension point for medical reports, the application is opened up to make it future-proof and customer-customizable. Customers or external companies, different from the developer of the medical application, can create and commercialize plug-ins that implement independent pluggable medical report components. This way, the invention enhances the reporting capabilities of the hosting application in a manner that eases integration in the hosting application and provides the customer the same look and feel as the rest of the application. Each plug-in can have a dedicated persistence structure, e.g. also facilitating access for statistical, data mining or reasoning purposes. The plugged sections can be used in different report structures to make a correct mix of format, multi-media content and text sections for each type of report. The enhanced configuration capabilities are provided by the plug-in section itself. For each plug-in section, a specific logic can be foreseen to render the section in a rendered document (pdf, html), suitable for later consultation or distribution.

The method according to the invention shall typically be computer-implemented on a system or platform with client-server architecture. The medical images, reports and tasks are maintained centrally or distributed on one or more servers. Users typically access their task lists and consult images or reports stored in the system via a client device. A data processing system or computing device that is operated according to the present invention can include a workstation, a server, a laptop, a desktop, a hand-held device, a mobile device, a tablet computer, or other computing device, as would be understood by those of skill in the art.

The data processing system or computing device can include a bus or network for connectivity between several components, directly or indirectly, a memory or database, one or more processors, input/output ports, a power supply, etc. One of skill in the art will appreciate that the bus or network can include one or more busses, such as an address bus, a data bus, or any combination thereof, or can include one or more network links. One of skill in the art additionally will appreciate that, depending on the intended applications and uses of a particular embodiment, multiple of these components can be implemented by a single device. Similarly, in some instances, a single component can be implemented by multiple devices.

The data processing system or computing device can include or interact with a variety of computer-readable media. For example, computer-readable media can include Random Access Memory (RAM), Read Only Memory (ROM), Electronically Erasable Programmable Read Only Memory (EEPROM), flash memory or other memory technologies, CDROM, digital versatile disks (DVD) or other optical or holographic media, magnetic cassettes, magnetic tape, magnetic disk storage or other magnetic storage devices that can be used to encode information and can be accessed by the data processing system or computing device.

The memory can include computer-storage media in the form of volatile and/or nonvolatile memory. The memory may be removable, non-removable, or any combination thereof. Exemplary hardware devices are devices such as hard drives, solid-state memory, optical-disc drives, or the like. The data processing system or computing device can include one or more processors that read data from components such as the memory, the various I/O components, etc.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A method of operating a data processing system for diagnostic workflow management with generation of sectional reports (200), **CHARACTERIZED IN THAT** said method comprises defining an extension point for report sections enabling users to create one or more independent pluggable components each of which implements a user-configurable report section (104, 204, 306, 406, 502).

2. A method according to claim 1, wherein said extension point represents a public interface comprising a title for configuring a title of a report section, a display visualisation for configuring visualization of said report section on a display, and a report visualisation for configuring visualization of said report section in a report.

3. A method according to claim 1 or claim 2, wherein said report section represents one or more of:
- a questionnaire (104, 204);
- a still image (306, 406, 502);
- moving images.

4. A data processing system comprising means for carrying out the method of any of claims 1 to 3.

5. A computer program comprising software code adapted to perform the method of any of claims 1 to 3.

6. A computer readable storage medium comprising the computer program of claim 5.
